# EUROPEAN PATENT APPLICATION

(11) **EP 2 494 964 A1**
(43) Date of publication of application: **05.09.2012**
(21) Application number: 12157993.2
(22) Date of filing: 02.03.2012
(51) Int. Cl.: A61K 9/20, A61K 31/41, A61K 31/4422, A61K 45/06, A61K 9/14, A61P 9/12

(54) **Combinations of valsartan and amlodipine**

(30) Priority: 03.03.2011 TR 201102067
(71) Applicant: Sanovel Ilaç Sanayi Ve Ticaret Anonim Sirketi, 34460 Sariyer/Istanbul (TR)
(72) Inventor: CIFTER, Ümit, 34398 Istanbul (TR); TÜRKYILMAZ, Ali, 34398 Istanbul (TR); SAYDAM, Mehtap, 34398 Istanbul (TR); TOMBAYOGLU, Vildan, 34398 Istanbul (TR)
(74) Representative: Sevinç, Erkan

(57) **Abstract**

The present invention provides pharmaceutical compositions suitable for direct compression into tablets, characterized by comprising valsartan or a pharmaceutically acceptable salt thereof with a mean particle size (d50) in the range of 15 to 60 µm and amlodipine or a pharmaceutically acceptable salt thereof with a mean particle size (d50) in the range of 2 to 20 µm. The invention also relates to a novel method of preparing pharmaceutical tablets involving direct compression of the composition according to the present invention.

## Description

### Field of Invention

The present invention relates to novel compositions suitable for direct compression, comprising combinations of valsartan or pharmaceutically acceptable salts thereof and amlodipine or pharmaceutically acceptable salts thereof. The invention further relates to a method for direct compression of the compositions into pharmaceutical tablets.

### Background of Invention

Valsartan is a specific angiotensin II receptor antagonist, which selectively and competitively blocks AT1-receptors and mediates the vasopressor and aldosterone affects of angiotensin II. Its chemical designation is N-(1-oxopentyl)-N-[[2'-(1H-tetrazol-5-yl)-[1,1'-biphenyl]-4-yl]methyl]-L-valine with the following chemical structure of Formula 1.

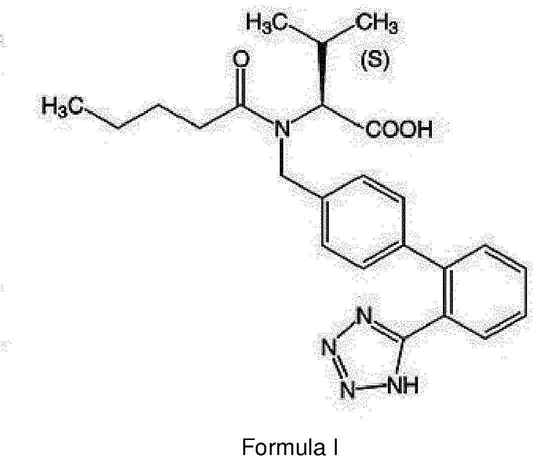

The valsartan molecule is used in treating hypertension. Valsartan is already available in 80, 160 and 320 mg tablets for oral administration.

Amlodipine is an orally-administered calcium channel blocker. Its chemical designation is (RS)-3-ethyl-5-methyl-2-(2-aminoethoxymethyl)-4-(2-chlorophenyl)-6-methyl-1,4-dihydropyridine-3,5-dicarboxylate with the following chemical structure of Formula II.

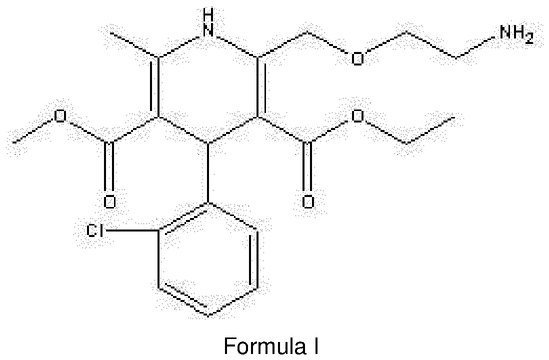

Amlodipine is presently available in the form of orally-administrable 5 to 10 mg tablets.

There have been various applications present in the patent relation for the combinations of valsartan and amlodipine.

The patent application W02007022113 discloses a solid formulation composed of valsartan, amlodipine and excipients.

The patent application W02011001440 discloses a composition composed of valsartan with mannitol and povidone. According to that application, it is also possible to add hydrochlorothiazide or amlodipine into the formulation.

The patent application US20110028456 claims a composition made of an active agent such as valsartan and/or amlodipine among the others, with a water-soluble or water-insoluble polymer.

The patent application W003097045 discloses a composition and kit of parts comprising valsartan, hydrochlorothiazide and amlodipine.

The patent application W02010081824 discloses a composition comprising a group from which enantiomerically-pure (S) and valsartan is selected.

One of the conventional tablet production methods is the direct compression method. While the production via direct compression has various difficulties, it is economic and has a high process speed. Production based on the direct compression method is affected by mixing the active agent(s) and excipient(s) used in the tablet formulation and then compressing this mixture under a properly-selected force level. The particle size of active agents and excipients is quite important in terms of the direct compression process. The causes that complicate this method are the lower amounts of active agents used, and the poor flowability and compressibility features of granules and particles.

Another important point in producing tablets is uniformly mixing the active agents that compose a formulation. It is thus ensured that any dosage form comprises the desired amount of active agent. In order to ensure a uniform mixing and to prevent any particle segregation, the difference in the particle size of the powders, pellets, and granules should not be large or should be kept within certain proportions. On the other hand, although the required particle size for direct compression does vary among the active agents and excipients, it must be kept above certain limits. If this size is not at a desired level, the direct compression process cannot be thoroughly achieved. Additionally, it should particularly be ensured that the particle size does not affect the solubility and dissolution rate parameters. Taking all these points into consideration, it may be concluded that the direct compression method for producing combinations of valsartan and amlodipine has significant difficulties. It is hereby preferred to achieve a uniform mixing and to prepare this mixture by means of an economic method such as direct compression which is advantageous in terms of the process speed.

In result, a improved and modified formulations, which can be used by hypertension patients are needed in the relevant art.

### Objects of the Invention

The present invention provides an easily-administrable combination compositions of valsartan and amlodipine, eliminating all aforesaid problems and bringing additional advantages to the relevant prior art.

Accordingly, the main object of the present invention is to obtain a combinations and compositions which have desired degree of uniformity in terms of its ingredients.

Another object of the present invention is to obtain an efficient and stable compositions for use in the treatment of hypertension.

A further object of the present invention is to obtain a combination of valsartan and amlodipine with proper particle sizes, which allows both to conduct a direct compression process, and to achieve a uniform mixing.
Yet a further object of the present invention is to obtain a combination of valsartan and amlodipine having desired levels of bioavailability.

Still another object of the present invention is to obtain a combination of valsartan and amlodipine having desired levels of dissolution rate and solubility.

### Detailed Description of the Invention

A novel pharmaceutical formulation has been developed to carry out all objects, referred to above and to emerge from the following detailed disclosure. The present invention provides also a novel method of producing pharmaceutical tablets by direct compression of the formulation above.

The novelty as referred above is realized with valsartan or pharmaceutically acceptable salts thereof with a mean particle size (d50) within the range of 15 to 60 µm and amlodipine or pharmaceutically acceptable salts thereof with a mean particle size (d50) within the range of 2 to 20 µm. Amlodipine, in this context, is preferably a besylate salt of amlodipine.

In a preferred embodiment according to the present invention, the mean particle size (d50) of valsartan is 20 to 50 µm and more preferably 25 to 45 µm.

In another preferred embodiment according to the present invention, the mean particle size (d50) of amlodipine is 3 to 15 µm and more preferably 5 to 10 µm.

According to the preferred embodiments, the proportion of the mean particle size (d50) of valsartan to the mean particle size (d50) of amlodipine may be 0.75 to 30, preferably 1 to 17, and more preferably 2 to 10.

The tablets according to the present invention, can be obtained by a direct compression method as a result of applying a force of 20 to 50 kN, preferably 25 - 45 kN, and more preferably 30 to 37 kN.

The combinations which are sepecifically designed for direct compression may comprise at least an excipient, selected from the group consisting of diluents, binders, disintegrants, glidants, lubricants and specific mixtures thereof.
In yet another preferred embodiment, said diluents may be selected from the group consisting of dextrose, magnesium carbonate, magnesium oxide, sucrose, cellulose acetate, maltodextrin, lactose, microcrystalline cellulose, starch, mannitol, calcium phosphate anhydrate, calcium phosphate dihydrate, calcium phosphate trihydrate, dibasic calcium phosphate, glucose and suitable mixtures thereof.

The aforesaid binders may suitably be selected from the group of hydroxyethyl cellulose (HEC), chitosan, carbomer, carrageenan, pectin, collagen, guar gum, gum tragacanth, shellac, carnauba wax, cetyl alcohol, stearic acid, polyamide, hydrogenated castor oil, alginic acid, beeswax, methacrylic acid/ethyl acrylate copolymer, dimethyl amino ethyl methacrylate/butyl methacrylate/metal methacrylate copolymer, hydroxypropyl methyl cellulose acetate succinate, gelatin, methacrylate derivatives, paraffin, polyvinyl acetate, cellulose acetate, cellulose phthalate, cellulose propionate, cellulose butyrate, cetostearyl alcohol, polymethacrylate, glyceryl behenate, polyvinylpyrrolidone (povidone), hydroxypropyl methyl cellulose (HPMC), hydroxypropyl cellulose (HPC), carboxymethyl cellulose (CMC), methyl cellulose (MC), ethyl cellulose and similar cellulose derivatives, polyethylene oxide, gelatin, and mixtures thereof.

In a further aspect of the present invention, said disintegrants may be selected from the group of croscarmellose sodium, sodium starch glycolate, crospovidone, starch (e.g. corn, potato), alginic acid, and mixtures thereof.
Glidants as referred in this context may be selected from the group of colloidal silicone dioxide, talk, aluminum silicate, and magnesium silicate.Lubricants as referred herein are selected from the group of glyceryl behenate, glyceryl monostearate calcium stearate, magnesium stearate, mineral oil, sodium stearyl fumarate, talk, polyethylene glycol, and stearic acidAccording to a further aspect of the present invention there is provided a novel formulation based on the composition as provided hereinabove. Said pharmaceutical formulation is consisting of the following ingredients:
a. valsartan or a pharmaceutically acceptable salt thereof at 5 - 33% by weight,
b. amlodipine or a pharmaceutically acceptable salt thereof at 0.3-1 % by weight,
c. pregelatinized starch at 10 - 60% by weight,
d. microcrystalline cellulose at 10 - 27% by weight,
e. colloidal silicone dioxide at 0.25 - 3% by weight,
f. magnesium stearate at 0.25 - 1.5% by weight,
g. coating layer at 0.1 - 5 % by weight
wherein valsartan and amlodipin have the specific d50 particle size arrangements as defined in the foregoing description.

According to another aspect of the present invention there is provided a method for preparing the pharmaceutical tablets according to the present invention, the method basically comprises the steps of:
a. adding excipients into a mixture of valsartan and amlodipine and mixing the resulting mixture, wherein valsartan has a mean particle size (d50) in the range of 15 to 60 µm and amlodipine has a mean particle size (d50) in the range of 2 to 20 µm,
b. directly compressing the mixture into tablets, and
c. film-coating the tablets obtained.

The film coating step may provide, for instance a coating layer of 0.1-5% by weight of the overall composition.

By virtue of the modified particle sizes of the active ingredients, as well as the specifically designed excipients, stable combination formulations of amlodipine and valsartan are obtained, which have surprisingly good in bioavailability, content uniformity, and dissolution rate. The inventors particularly report that flowability and compressibility of the powder formulations are particularly improved that is attributed to the specific particle size arrangement of valsartan and amlodipine. It was noted that this particular effect was not achievable with conventional sieving practices using for instance a wide range of 10 to 100 meshes. Thanks to compression force and particle sizes of the active ingredients are achieved the present invention.

In the tablet formulations according to the present invention, valsartan may be used in amounts of 80, 160 to 320 mg, whereas amlodipine is usable in amounts of 5 to 10 mg, which however must be deemed illustrative, but not limiting.

The protection scope of the present invention is set forth in the appended claims and cannot be restricted to the illustrative disclosures given above, under the detailed description.

## Claims

1. A pharmaceutical composition suitable for direct compression, **characterized by** comprising valsartan or a pharmaceutically acceptable salt thereof with a mean particle size (d50) in the range of 15 to 60 µm and amlodipine or a pharmaceutically acceptable salt thereof with a mean particle size (d50) in the range of 2 to 20 µm.

2. The pharmaceutical composition according to Claim 1, wherein the mean particle size (d50) of valsartan is 20 to 50 µm and preferably 25 to 45 µm.

3. The pharmaceutical composition according to claims 1 and 2, wherein the mean particle size (d50) of amlodipine is 3 to 15 µm and preferably 5 to 10 µm.

4. The pharmaceutical composition according to any of the preceding claims, wherein amlodipine is amlodipine besylate.

5. The pharmaceutical composition according to any of the preceding claims, wherein the proportion of the mean particle size (d50) of valsartan to the mean particle size (d50) of amlodipine is 0.75 to 30, preferably 1 to 17, and more preferably 2 to 10.

6. The pharmaceutical composition according to any of the preceding claims, wherein said tablet is obtained by direct compression method as a result of applying a force of 20 to 50 kN, preferably 25 - 45 kN, and more preferably 30 to 37 kN.

7. The pharmaceutical composition according to any of the preceding claims, further comprising at least one excipient.

8. The pharmaceutical composition according to any of the preceding claims, wherein said excipient is selected from the group consisting of diluting agents, binders, disintegrants, glidants, lubricants and mixtures thereof.

9. The pharmaceutical composition according to any of the preceding claims, wherein said diluent comprises at least one or a mixture of dextrose, magnesium carbonate, magnesium oxide, sucrose, cellulose acetate, maltodextrin, lactose, microcrystalline cellulose, starch, mannitol, calcium phosphate anhydrate, calcium phosphate dihydrate, calcium phosphate trihydrate, dibasic calcium phosphate, and glucose.

10. The pharmaceutical composition according to any of the preceding claims, wherein said binder comprises at least one or a mixture of hydroxyethyl cellulose (HEC), chitosan, carbomer, carrageenan, pectin, collagen, guar gum, gum tragacanth, shellac, carnauba wax, cetyl alcohol, stearic acid, polyamide, hydrogenated castor oil, alginic acid, beeswax, methacrylic acid/ethyl acrylate copolymer, dimethyl amino ethyl methacrylate/butyl methacrylate/metal methacrylate copolymer, hydroxypropyl methyl cellulose acetate succinate, gelatin, methacrylate derivatives, paraffin, polyvinyl acetate, cellulose acetate, cellulose phthalate, cellulose propionate, cellulose butyrate, cetostearyl alcohol, polymethacrylate, glyceryl behenate, polyvinylpyrrolidone (povidone), hydroxypropyl methyl cellulose (HPMC), hydroxypropyl cellulose (HPC), carboxymethyl cellulose (CMC), methyl cellulose (MC), ethyl cellulose and similar cellulose derivatives, polyethylene oxide, and gelatin.

11. The pharmaceutical composition according to any of the preceding claims, wherein said disintegrant comprises at least one or a mixture of croscarmellose sodium, sodium starch glycolate, crospovidone, starch, and alginic acid.

12. The pharmaceutical composition according to any of the preceding claims, wherein said glidant comprises at least one or a mixture of colloidal silicone dioxide, talk, aluminum silicate, and magnesium silicate.

13. The pharmaceutical composition according to any of the preceding claims, wherein said lubricant comprises at least one or a mixture of glyceryl behenate, glyceryl monostearate, calcium stearate, magnesium stearate, mineral oil, sodium stearyl fumarate, talk, polyethylene glycol, and stearic acid.

14. A pharmaceutical composition according to any of the preceding claims, consisting of:
a. valsartan or a pharmaceutically acceptable salt thereof at 5-33% by weight,
b. amlodipine or a pharmaceutically acceptable salt thereof at 0.3-1% by weight,
c. pregelatinized starch at 10 - 60% by weight,
d. microcrystalline cellulose at 10 to 27% by weight,
e. colloidal silicone dioxide at 0.25 to 3% by weight,
f. magnesium stearate at 0.25 to 1.5% by weight.
g. coating layer at 0.1 - 5 % by weight.

15. A method for preparing pharmaceutical tablets, comprising the steps of
a. adding excipients into a mixture of valsartan and amlodipine and mixing the resulting mixture, wherein valsartan has a mean particle size (d50) in the range of 15 to 60 µm and amlodipine has a mean particle size (d50) in the range of 2 to 20 µm,
b. directly compressing the mixture into tablets, and
c. film-coating of the tablets thus obtained.

16. A method according to claim 15 wherein the excipients are selected from the group consisting of diluents, binders, disintegrants, glidants, lubricants and mixtures thereof.

17. The pharmaceutical composition according to any of the preceding claims for use in preventing or treating hypertension in mammalians and particularly in humans.
